# EUROPÄISCHE PATENTSCHRIFT

(11) **EP 1 766 420 B1**
(45) Veröffentlichungstag und Bekanntmachung des Hinweises auf die Patenterteilung: **02.03.2011**
(21) Anmeldenummer: 05769605.6
(22) Anmeldetag: 12.07.2005
(51) Int. Cl.: G01N 35/10, G01N 33/49

(54) **EINRICHTUNG UND VERFAHREN ZUR AUTOMATISCHEN UNTERSUCHUNG VON BLUTPROBEN**
DEVICE AND METHOD FOR AUTOMATICALLY ANALYSING BLOOD SAMPLES
DISPOSITIF ET PROCEDE D'ANALYSE AUTOMATIQUE D'ECHANTILLONS DE SANG

(30) Priorität: 12.07.2004 DE 102004033654
(43) Veröffentlichungstag der Anmeldung: 28.03.2007
(73) Patentinhaber: Kratzer, Michael, 80799 Munchen (DE); VDG von der Goltz GmbH, 83370 Seeon (DE)
(72) Erfinder: KRATZER, Michael, 80799 München (DE); FREIHERR VON DER GOLTZ, Volker, 83370 Seeon (DE)
(74) Vertreter: von Puttkamer · Berngruber
(86) Internationale Anmeldenummer: PCT/DE2005/001228
(87) Internationale Veröffentlichungsnummer: WO 2006/005326

(56) Entgegenhaltungen:
- EP-A- 0 522 256
- EP-A- 0 812 570
- DE-A1- 2 131 423

## Beschreibung

Die vorliegende Erfindung betrifft eine Einrichtung und ein Verfahren zur automatischen Untersuchung von Blutproben.

Eine derartige Einrichtung geht aus der EP 0 522 256 B1 hervor. Dabei werden einem Prüfkopf gleichzeitig aus einer Magazineinrichtung Vorratsgefäße mit Blut und aus einer weiteren Magazineinrichtung Aperturhalter zugeführt. Der Prüfkopf weist einen Kapillarenhalter auf, an dem wenigstens eine in ein Vorratsgefäß einführbare Kapillare befestigt ist. Ferner besitzt der Prüfkopf ein Kopfteil, das durch Relativbewegung zum Kapillarenhalter zwischen einer ersten Position, in der ein Aperturhalter auf dem Kapillarenhalter angeordnet wird und einer zweiten Position bewirkt wird, in der der Aperturhalter zwischen dem Kapillarenhalter und dem Kopfteil so gehalten wird, dass über die in ein Vorratsgefäß eintauchende Kapillare Blut aus dem Vorratsgefäß durch die Kapillare und die Apertur des Aperturhalters hindurchführbar ist. Nach der Vornahme einer Messung werden der benutzte Aperturhalter und das benutzte Vorratsgefäß nach Bewegen des Kopfteiles in die erste Position aus dem Prüfkopf entfernt. Danach wird der Prüfkopf durch eine weitere Vorrichtung gereinigt.

Aus der DE 21 31 423 A1 geht eine gattungsgemäße Einrichtung für die Untersuchung von Blutserum hervor, die eine Entnahmestation, in der eine Probe aus einem Vorratsgefäß mit einer Entnahmeeinrichtung entnehmbar ist, eine Messstation, in der die entnommene Probe durch eine Apertur in einem Messteil hindurchführbar ist, wobei das Messteil mit der Entnahmevorrichtung verbunden ist, eine Aufnahmestation, in der als Wegwerwrfteile ausgebildete Messteile bereitstellbar und mit der Entnahmevorrichtung verbindbar sind, und eine Einrichtung zum Bewegen der Entnahmevorrichtung zwischen der Entnahmestation, der Messstation und der Aufnahmestation umfasst.

Die Aufgabe der vorliegenden Erfindung besteht darin, eine derartige Einrichtung zur automatischen Untersuchung von Blutproben wesentlich zu vereinfachen, wobei ferner kostengünstigere Untersuchungen ermöglicht werden sollen. Durch die Erfindung wird auch ein Verfahren zur Durchführung der Untersuchungen bereitgestellt.

Diese Aufgabe wird durch eine Einrichtung zur automatischen Untersuchung von Blutproben mit den Merkmalen des Patentanspruches 1 sowie durch ein Verfahren mit den Merkmalen des Patentanspruches 32 gelöst.

Der wesentliche Vorteil der vorliegenden Erfindung besteht darin, dass die vorliegende Einrichtung sehr viel einfacher aufgebaut ist und daher auch wirtschaftlicher arbeiten kann, als die bekannten vergleichbaren Einrichtungen. Es werden vorteilhafter Weise kostengünstigere Untersuchungen ermöglicht. Dies ist im wesentlichen darauf zurückzuführen, dass lediglich die als Einweg- bzw. Wegwerfteile ausgebildeten Messteile mit dem Aperturhalter aus einer Magazineinrichtung zugeführt werden. Die Zuführung der Blut-Vorratsgefäße aus einer weiteren Magazineinrichtung kann wegen der speziellen Ausgestaltung sowohl des Kopfteiles als auch des Messteiles der vorliegenden Erfindung entfallen. Dadurch, dass die erfindungsgemäße Einrichtung im wesentlichen so konzipiert ist, dass sie verschiedene Stationen, nämlich eine Blut-Entnahmestation, eine Messstation, sowie gegebenenfalls eine Zuführstation für eine die Plättchenbildung oder die Koagulation beeinflussende Substanz (z.B. ADP), eine Waschstation und eine Entsorgungsstation in einer vorgegebenen Reihenfolge anfährt, können das bei der EP 0 522 256 B1 vorhandene Kopfteil sowie der Prüfkopf, die beide sehr kompliziert aufgebaut sind, entfallen. Die aufwändigen Ansteuerungen und Bewegungen dieser Teile sind ebenfalls entbehrlich.

Vorteilhafte Ausgestaltungen der Erfindung gehen aus den Unteransprüchen hervor.

Im folgenden werden die Erfindung-und deren Ausgestaltungen im Zusammenhang mit den Figuren näher erläutert. Es zeigen:
Figur 1 in schematischer Darstellung die erfindungsgemäße Einrichtung;
Figur 2 eine Ansicht der Einrichtung der Figur 1 von oben; und
Figur 3 die Seitenansicht eines Messteiles mit einem Aperturhalter sowie eines in das Messteil eingefahrenen Kopfteiles; und
Figur 4 ein weiteres bevorzugtes Messteil.

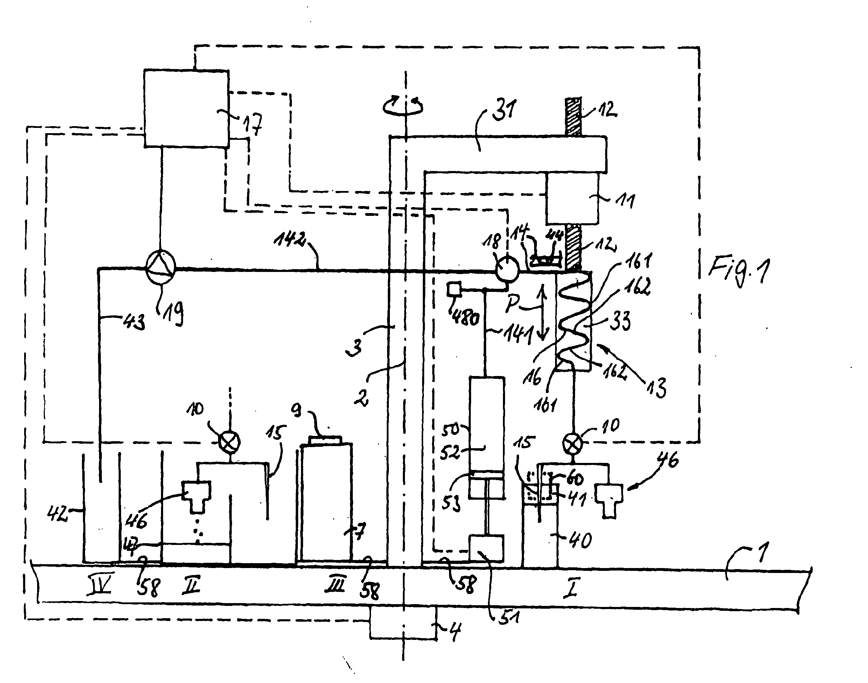

Gemäß Figur 1 umfasst die vorliegende Einrichtung zur automatischen Untersuchung von Blutproben im wesentlichen auf einer Arbeitsplatte 1 oder dergleichen eines Messplatzes einen um eine Drehachse 2 zwischen mehreren Stationen I, II und III sowie gegebenenfalls weiteren Stationen IV und V verschwenkbaren Dreharm 3.

Dabei handelt es sich bei der ersten Station I um eine Blut-Entnahmestation, bei der Blut aus einem Vorratsgefäß 40 entnommen wird.

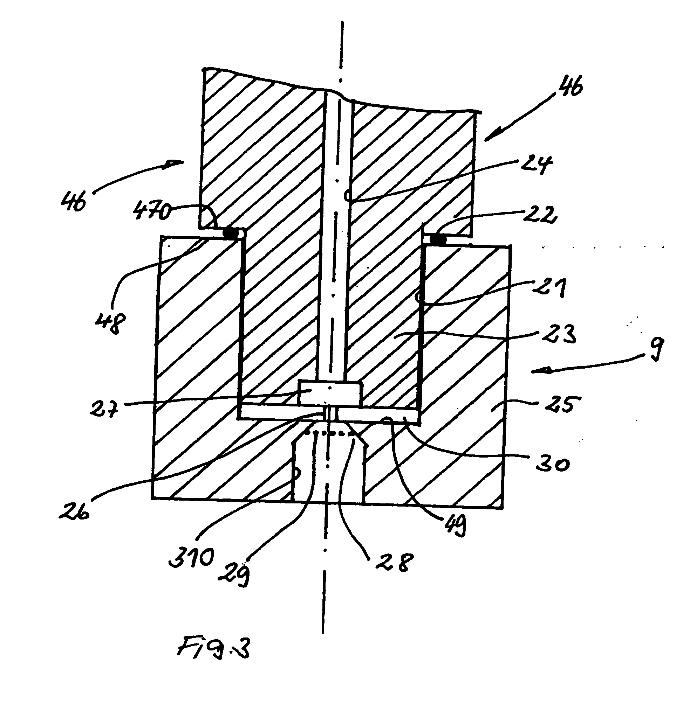

Die Station II ist eine Messstation, bei der aus dem Vorratsgefäß 40 entnommenes Blut über ein Kopfteil 46 in der später näher erläuterten Weise zur Ausführung einer Messung durch die Apertur eines Aperturhalters eines Messteiles 9 (Figur 3) hindurch geführt wird, wobei das Messteil 9 und das Kopfteil 46 in der Messstation II dicht miteinander verbunden werden.

In der Aufnahmestation III werden aus einer schematisch dargestellten Magazineinrichtung 7 die den Aperturhalter 30 (Figur 3) jeweils umfassenden Messteile 9, die als Einmal- bzw. Wegwerfteile ausgebildete sind, aufeinanderfolgend zur Ankoppelung an das kopfteil 46 bereit gestellt.

Ferner können eine Zugabestation für eine die Plättchenbildung oder die Koagulation beeinflussende Substanz und eine Waschstation vorgesehen sein, wie dies später erläutert wird.

Der Dreharm 3 wird mit der Hilfe eines schematisch dargestellten Antriebes 4 in Drehung versetzt. Bei dem Antrieb 4 handelt es sich vorzugsweise um einen Schrittmotor, der zum Anfahren der einzelnen Stationen exakt ansteuerbar ist.

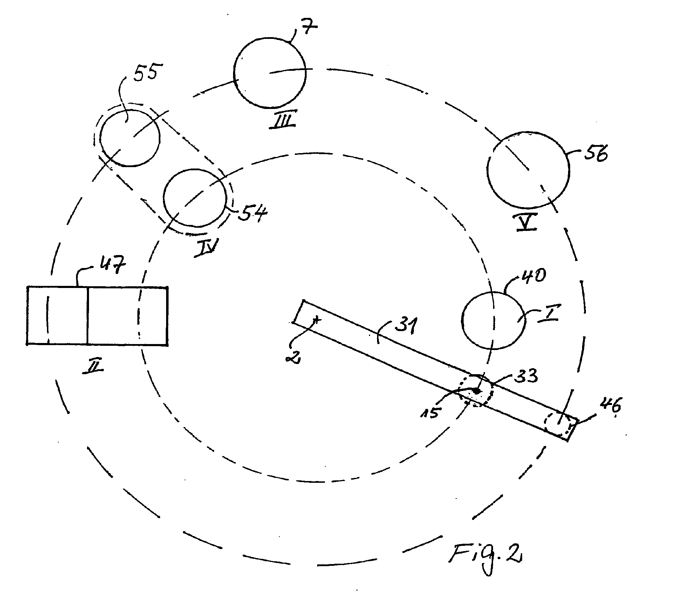

Es wird darauf hingewiesen, dass die genannten Stationen gemäß den Figuren 1 und 2 bevorzugt auf einem Kreisbogen liegen. Ebenso gut ist es jedoch denkbar, dass diese Stationen beispielsweise geradlinig angeordnet sind und dass der Dreharm 3 linear oder in x-y-Richtung bewegt wird.

In der aus der Figur 1 ersichtlichen Weise ist am Auslegerteil 31 des Dreharmes 3 ein Antrieb 11 befestigt, mit dessen Hilfe ein Halteteil 13 vorzugsweise senkrecht zur Ebene der Arbeitsplatte 1 auf- und abwärts bewegbar ist, wie dies durch den Pfeil P angedeutet ist. Zweckmäßigerweise handelt es sich bei dem Antrieb 11 um einen an sich bekannten Mechanismus, mit dem eine Gewindestange 12 in der Richtung des Pfeiles P bewegbar ist, wobei an der Gewindestange das Halteteil 13 befestigt ist.

Das mit der Hilfe des Antriebes 11 in der Richtung des Pfeiles P hin- und her bewegbare Halteteil 13 umfasst ein Nadelteil 15, das an der der Arbeitsplatte 1 zugewandten Seite angeordnet ist und an seinem der Arbeitsplatte 1 abgewandten Ende mit einer wenigstens teilweise schlangen- oder spiralförmig verlaufenden und am Halteteil 13 gehaltenen Rohrleitung 16 verbunden ist, die an ihrer dem Nadelteil 15 abgewandten Seite mit einer Leitung 14 verbunden ist, bei der es sich vorzugsweise um einen Schlauch handelt. Die Rohrleitung 16 und das Nadelteil 15 sind vorzugsweise innenseitig und/oder außenseitig hydrophob ausgebildet, sodass eingesaugte Blutbestandteile nicht an den entsprechenden Innen- und/oder Außenwandungen anhaften. Vorzugsweise bestehen die Rohrleitung 16 und/oder das Nadelteil 15 aus einem innenseitig mit einer hydrophoben oder oleophoben Beschichtung versehenen Material, insbesondere Metallmaterial. Bei der genanten Beschichtung kann es sich vorzugsweise um eine an sich bekannte Sol-Gel-Nanokomposit-Schicht oder um eine teflonartige Fluorpolymer-Schicht handeln. Bei dem Nadelteil 15 kann eine derartige Beschichtung auch außenseitig vorgesehen sein.

Am Halteteil 13 ist vorzugsweise vom Nadelteil 15 beabstandet ein Kopfteil 46 angeordnet, das zur wahlweisen Verbindung mit einem Messteil 9 dient, wie dies später näher erläutert wird. Das Nadelteil 15 und das Kopfteil 46 sind mit einem Ventil 10 verbunden, das durch eine Steuereinheit 17 angesteuert wird und wahlweise eine Verbindung zwischen dem Nadelteil 15 und der Rohrleitung 16 oder zwischen dem Kopfteil 46 und der Rohrleitung 16 herstellen kann.

Die Leitung 14 führt zu einem durch die Steuereinheit 17 ansteuerbaren Ventil 18, das eine Verbindung zu einer Leitung 141 herstellen kann, die mit einem Aufnahmereservoir 50, vorzugsweise einer Kolben/Zylindereinheit verbunden ist. Diese Kolben/Zylindereinheit wird durch einen Antrieb 51 betätigt, der wiederum von der Steuereinheit 17 angesteuert wird. Das Ventil 18 kann ferner eine Verbindung zwischen der Leitung 14 und einer Leitung 142 herstellen, die mit einer Pumpe 19 verbunden ist, die über eine Leitung 43 zu einem Behältnis 42 führt, in dem sich eine Spülflüssigkeit, vorzugsweise NaCl befindet.

Besonders bevorzugt werden in der Station III von der Magazineinrichtung 7 aufeinanderfolgend Messteile 9 in die Bewegungsbahn des Dreharmes 3 zugeführt.

Das Halteteil 13 umfasst in der aus der Figur 1 ersichtlichen Weise neben der mit dem Nadelteil 15 verbundenen Rohrleitung 16 auch eine schematisch angedeutete Heizeinrichtung 33, die insbesondere die Form eines metallischen Heizkernes besitzt, an dem die Rohrleitung 16 schlangenförmig angeordnet verläuft oder auf den sie spiralförmig aufgewickelt ist. Die in der Figur 1 gezeigte schlangenförmige Anordnung hat den Vorteil, dass in aufeinanderfolgenden Schleifen die Ober- und Unterseiten der über eine Biegung 161 jeweils verbundenen Abschnitte 162 jeweils umgekehrt werden, sodass das Blut beim Durchlaufen der Abschnitte quasi "umgestülpt" bzw. "umgekippt" wird. Eine Sedimentation des Blutes wird daher weitgehendst bzw. vollständig vermieden. Weitere Heizeinrichtungen können für die Messteile 46 und/oder das Nadelteil 15 vorgesehen werden, wie dies später erläutert wird.

Im folgenden wird im Zusammenhang mit der Figur 3 eine bevorzugte Ausführungsform des Messteiles 9 sowie des Kopfteiles 46 erläutert . Das Kopfteil 46 besitz einen unteren Bereich 23 der dicht in eine Ausnehmung 21 des Messteiles 9 eingeführt werden kann. Die Abdichtung zwischen dem Kopfteil 46 und dem Messteil 9 wird dabei vorzugsweise durch eine O-Dichtung 22 erreicht, die an einer Schulter 47 des unteren Bereiches 23 angeordnet ist und beim Einfahren des unteren Bereiches 23 in die Ausnehmung 21 an einer Fläche 48 des Randbereiches der Ausnehmung 21 dicht zur Anlage gelangt.

Im Kopfteil 46 verläuft eine Durchgangsöffnung 24, die die Form einer Kapillare aufweisen kann und oberseitig mit der Rohrleitung 16 über das Ventil 10 verbunden ist. Die Durchgangsöffnung 24 reicht bis zu einem eine Vorkammer 27 bildenden Raum am unteren Ende des Bereiches 23. An der unteren Bodenfläche 49 der Ausnehmung 21 befindet sich in an sich bekannter Weise ein Aperturhalter 30 mit einer zur Durchgangsöffnung 24 ausgerichteten Apertur 26. Im Körperteil 25 des Messteiles 9 befindet sich hinter dem Aperturhalter 30 ein Raum, der eine Nachkammer 28 bildet und nach unten in einen Blutaustrittsraum 310 übergeht. Das Kopfteil 46 und das Messteil 9 sind mit der Hilfe nicht näher dargestellter Verriegelungsmechanismen durch Aufeinanderzubewegen aneinander dicht befestigbar und durch Voneinanderwegbewegen voneinander trennbar.

Beim Eintreten von Blut aus der Rohrleitung 16 in das Kopfteil 46 und in das Messteil 9 gelangt das Blut aus der Durohgangsöffnung 24 über die Vorkammer 27 in die Apertur 26 des Aperturhalters 30 und danach in die Nachkammer 28 und den Blutaustrittsraum 310. In der Station II kann dabei das aus dem Blutaustrittsraum 310 austretende Blut in einem Auffangbehälter 47 aufgefangen werden.

Im folgenden wird der Betrieb der vorliegenden Einrichtung näher erläutert. Dabei wird zunächst davon ausgegangen, dass alle Teile bzw. Elemente der vorliegenden Einrichtung, die mit Blut in Verbindung treten, gereinigt sind.

Zunächst wird der Dreharm 3 durch den Antrieb 4 zur Station I gefahren und wird der Antrieb 11 zum Absenken des Halteteiles 13 betätigt (Pfeil P). Wenn das Nadelteil 15 den Verschluss 41 des Vorratsgefäßes 40 durchstoßen hat und in das darin enthaltene Blut eintaucht, wird der Kolben 53 der Kolben/Zylindereinheit des Aufnahmereservoirs so durch den Antrieb 51 betätigt. Vorzugsweise handelt es sich bei dem Verschluss 41 um einen Stopfen, der vorzugsweise aus einem Gummimaterial besteht.

Um Sedimentationen im Vorratsgefäß 40 zu vermeiden, ist zweckmäßigerweise eine nicht näher dargestellte Vorrichtung vorgesehen, die das Vorratsgefäß 40 fortlaufend bewegt, invertiert oder verdreht.

Es wird darauf hingewiesen, dass in dem System der Rohrleitung 16 sowie der Leitungen 14, 141, 142, 43 zuvor eine neutrale Flüssigkeit enthalten ist, bei der es sich beispielsweise um eine über die Leitung 43 aus dem Behältnis 42 entnommene Spülflüssigkeit, zum Beispiel eine physiologische NaCl-Lösung, handeln kann. Zwischen dem eingesaugten Blut und der genannten Flüssigkeit befindet sich dann als Trennmedium die in der Figur 1 vergrößert dargestellte Luftblase 44 vorzugsweise in dem Bereich zwischen dem Ventil 18 und der Rohrleitung 33. Durch Betätigen der Pumpe 19 kann das eingesaugte Blut zweckmäßigerweise bis zur Vornahme der Messung fortlaufend hin- und herbewegt werden, so dass es ständig in Bewegung bleibt und nicht sedimentiert. Auf den Vorteil der schlangenförmig verlaufenden Rohrleitung 16 wurde bereits hingewiesen. Zum beschriebenen Einsaugen des Blutes stellt das Ventil 18 eine Verbindung zwischen der Rohrleitung 16 und der Leitung 141 her. Zum Hin- und Herbewegen des Blutes kann bei dieser Stellung des Ventils 18 der Kolben 53 im Zylinderraum 52 des Aufnahmereservoirs 50 standig hin und her bewegt werden. Das Blut wird dabei durch die Heizeinrichtung 33 fortlaufend auf die erforderliche Temperatur von z.B. 37°C gebracht.

Der Dreharm 3 wird nun zur Station III bewegt und entnimmt der Magazineinrichtung 7 ein Messteil 9. Zu diesem Zweck wird zunächst der Antrieb 4 betätigt, um den Dreharm 3 über die Magazineinrichtung 7 zu bewegen. Dann werden der Antrieb 11 betätigt und das Kopfteil 46 abgesenkt, bis dieses in der oben erläuterten Weise am bereit gestellten Messteil 9 dicht verriegelt wird. Nach der Aufnahme des Messteiles 9 wird der Antrieb 11 erneut betätigt, so dass das Kopfteil 46 und das damit verbundene Messteil 9 angehoben und von der Magazineinrichtung 7 entfernt werden. Es wird darauf hingewiesen, dass die Magazineinrichtung 7 auch eine andere Form aufweisen kann. Beispielsweise kann die Magazineinrichtung einen Bandförderer oder dergleichen umfassen, mit dem fortlaufend Messteile 9 zum Ort III befördert werden. Hier werden sie dann in der voranstehende Weise mit dem Kopfteil 46 verbunden.

Ansohließend wird der Dreharm 3 durch den Antrieb 4 zur Messstation II bewegt. Dort wird die eigentliche Messung ausgeführt. Dabei wird der Kolben 53 der Kolben/Zylindereinheit 50 so bewegt, dass Blut ständig durch die Apertur 26 des Messteiles 9 strömt und dieses durch den Blutaustrittsraum 31 verlässt. Dabei verbindet das Ventil 10 das Kopfteil 46 mit der Rohrleitung 16. Aus dem Messteil 9 austropfendes Blut wird in dem Auffangbehälter 47 aufgefangen. Während dieses Messvorganges wird ständig der Volumenstrom des Blutes durch Messen des Druckes mit einem Drucksensor 480 gemessen und über bekannte Verfahren wird die Bewegung des Kolbens 53 entsprechend gesteuert, um eine bestimmte Druck/Volumenstromkennlinie zu erzeugen. Hierbei wird ein Thrombus in der Apertur 26 ausgebildet. Anschließend wird das Kopfteil 46 durch Betätigen des Antriebes 11 in der oben erläuterten Weise aus dem Messteil 9 herausgezogen und wird das Messteil 9 z.B. in den Auffangbehälter 47 ausgeworfen.

Zum Spülen der Leitungen 16, 14 sowie des Nadelteiles 15 wird nach der Vornahme einer Messung und dem Ausstoßen des darin enthaltenen Blutes Spülmittel, das vorzugsweise dem Behältnis 42 durch Betätigen der Pumpe 19 entnommen wird, durch die genannten Leitungen bzw. Leitungsabschnitte und das Nadelteil 15 gepumpt, wobei das Ventil 10 entsprechend geschaltet ist. Aufgrund der beschriebenen hydrophoben oder olephoben Beschichtungen wird eine schnelle Reinigungswirkung erzielt. Zur Reinigung der Außenseite des Nadelteiles 15 kann dieses zu einer eigenen Spülstation IV durch Betätigen des Antriebes 4 verfahren werden, und in ein Spül- oder Reinigungsmittel enthaltenes Behältnis 54 (Figur 3) eingetaucht werden, wobei hierbei der Antrieb 11 betätigt wird.

Zur Reinigung des Messkopfes 46 wird bei entsprechender Stellung des Ventiles 10 Spülmittel durch den Messkopf 46 gepumpt. Zur Reinigung der Außenseite des Messkopfes 46 kann dieser in der Station IV vorzugsweise gleichzeitig mit dem Nadelteil 15 in das Reinigungs- oder Spülmittel eines weiteren behältnisses 55 (Figur 2) eingetaucht werden

In einer weiteren Station V kann, wie oben bereits erwähnt vorzugsweise ein der Magazineinrichtung 7 entnommenes Messteil 9 in ein Behältnis 56 (Figur 2) eingetaucht werden, das z.B. ADP oder ein anderes flüssiges Medium enthält, das beim Eintauchen in das poröse Material des Aperturhalters 30 und zur Wandung der Apertur 26 gelangt. Dieses Medium beeinflusst bei der Messung das Gerinnungs- bzw. Aggregationsverhalten des die Apertur 26 durchströmenden Blutes in einer vorbestimmten Weise.

Das Behältnis 42 und der Antrieb 51 mit der Kolben/Zylindereinheit 50 sind vorzugsweise über eine Haltevorrichtung 58 drehfest mit dem Arm 3 verbunden.

Gemäß Figur 1 kann vorzugsweise eine weitere am Halteteil 33 angeordnete Heizeinrichtung 60 vorgesehen sein, mit deren Hilfe das Nadelteil 15 erwärmbar ist. Dabei kann diese weitere Heizeinrichtung 60, die lediglich schematisch dargestellt ist, zweckmäßigerweise die Form eines das Nadelteil 15 teilweise umgebenden Heizkörpers oder eines Warmluftgebläses aufweisen. Eine entsprechende Heizeinrichtung kann auch für das Kopfteil 46 vorgesehen werden.

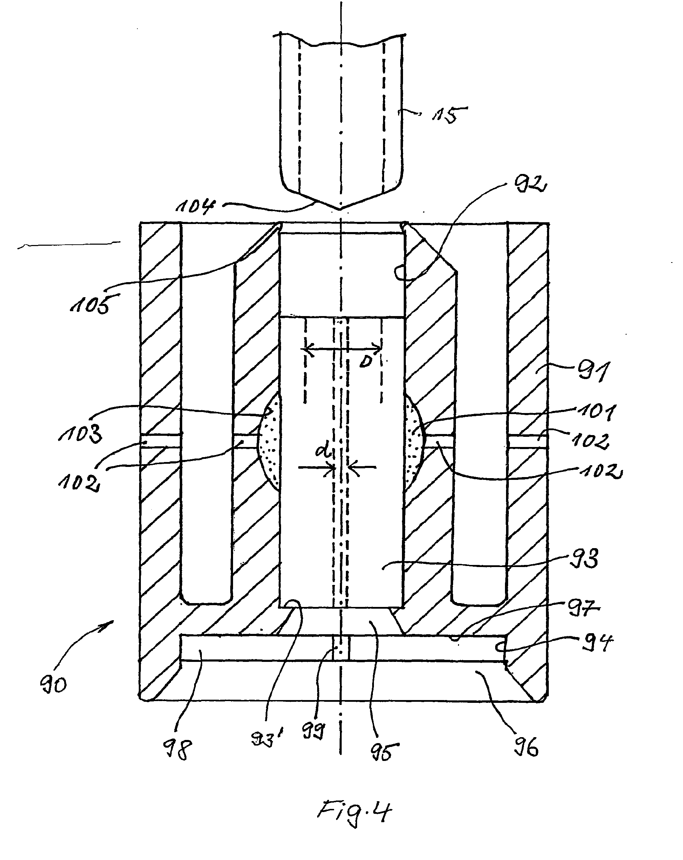

Im folgenden wird im Zusammenhang mit der Figur 4 eine weitere bevorzugte Ausführungsform eines Messteiles 90 erläutert, das direkt mit dem Nadelteil 15 verbindbar ist. Auf diese Weise können das Kopfteil 46 sowie das Ventil 10 entfallen. Das Messteil 90 besitzt in einem Körperteil 91 eine zentrale Öffnung 92, in die bzw. aus der das Nadelteil 15 bei Betätigung des Antriebes 11 einfahr- bzw. ausfahrbar ist. In der im Inneren der in Längsrichtung des Körperteiles 91 verlaufenden Öffnung 92, vorzugsweise einer Bohrung, befindet sich ein Röhrchen 93, das sich bis zu einer unteren Schulter 93' zwischen der Öffnung 92 und einer sich im Körperteil 91 an diese anschließende Vorkammer 95 erstreckt. Die Vorkammer 95 geht in einen erweiterten Raum 94 des Körperteiles 91 über, der sich wiederum in eine Nachkammer 96 des Körperteiles 91 öffnet, die als Blutaustrittsraum dient. An einer Schulter 97 zwischen der Vorkammer 95 und dem erweiterten Raum 94 liegt der Aperturhalter 98 an, derart, dass er eine diche Abtrennung zwischen der Vorkammer 95 und der Nachkammer 96 bewirkt. Die Apertur 99 des Aperturhalters 98 verbindet daher die Vorkammer 95 und die Nachkammer 96. Der Aperturhalter 98 kann aus einem porösen oder nicht porösen Material bestehen.

Das Nadelteil 15 weist an seiner der Bohrung 92 zugewandten Seite einen konischen Endbereich 104 auf der dicht in die Bohrung 92 einführbar ist. Zu diesem Zweck besitzt die öffnung 92 an ihrem oberen Ende ein ringförmiges Dichtungselement 105, das Form eines Dichtungswulstes oder einer O-Dichtung aufweisen kann. Es ist auch denkbar die Dichtung dadurch zu bewirken, dass der dem Nadelteil 15 zugewande Endbereich der Bohrung 92 entsprechend dem konischen Endbereich 104 zur dichten Aufnahme desselben konisch gestaltet wird.

Das Röhrchen 93 kann vorzugsweise entweder einen kreisringförmigen Querschnitt mit einem relativ großen Innendurchmesser D oder auch zur Nachbildung einer Kapillare mit einem entsprechend kleinen Innendurchmesser d besitzen. Vorzugsweise betragen der Außendurchmesser des Röhrchens 0,8 - 2,0 mm und der Innendurchmesser 150 - 500µm.

Vorzugsweise besteht das Körperteil 91 des als Wegwerf- bzw. Einmalteil ausgebildeten Messteiles 90 aus einem Kunststoffmaterial, wobei der Aperturhalter 98 an der Schulter 97 mit der Hilfe einer Ultra-Schall-Schweißung oder einer Klebung dicht befestigt sein kann.

Das Röhrchen 93 besteht vorzugsweise aus Edelstahl oder aus Fluor-Kunststoff (z.B. Tefzel ®). Es ist zweckmäßigerweise mit der Hilfe eines Klebemittels 101 am Innenumfang der Öffnung 92 befestigt, wobei das Klebemittel 101 über einen in Querrichtung des Körperteiles 91 verlaufenden Kanal 102 in eine als Klebemittelraum dienende Einbuchtung 103 injiziert werden kann, die sich in der Innenwand der Öffnung 92 befindet. Vorzugsweise ist die Einbuchtung zur Herstellung einer besonders festen und gleichförmigen Verbindung ringförmig ausgebildet.

Anstelle der erläuterten Verklebung kann das Röhrchen 93 auch durch eine Flächenpressung in der öffnung 92 fixiert sein.

Bei der Ausführung einer Messung wird zunächst über das Nadelteil 15 in der beschriebenen Weise Blut aus dem Reservoir 40 eingesaugt (Station I). Nach Verfahren des Dreharmes 3 zur Aufnahmestation III wird das Nadelteil 15 direkt in ein Messteil 90 der Magazineinrichtung 7 eingefahren und mit diesem dicht verbunden. Nach Bewegen des Armes 3 zur Messstation II wird dann das zuvor eingesaugte Blut vom Nadelteil 15 aus in die Öffnung 92 bzw in das Röhrchen 93 befördert. Es durchströmt dann die Vorkammer 95 und die Apertur 99 und qelanqt von dort aus in die Nachkammer 96.

Es wird darauf hingewiesen, dass die Ausrichtung des Nadelteiles 15 und der Apertur 99 sowie des sich ergebenden Blutflusses bei der Messung anstelle der dargestellten Richtung von oben nach unten auch umgekehrt oder auch horizontal sein kann. Auch andere Ausrichtungen sind denkbar.

Bei einer alternativen Ausführungsform kann das Röhrchen 93 entfallen, wobei dann die öffnung 92 einen entsprechenden Innendurchmesser von 200-1000 *µ*m besitzt.

## Patentansprüche

1. Einrichtung zur automatischen Untersuchung von Blutproben, mit
- einer Entnahmestation (I), in der Blut aus einem Vorratsgefäß (40) mit einer Entnahmevorrichtung entnehmbar ist,
- einer Messstation (II), in der entnommenes Blut durch eine Apertur (26; 99) eines in einem Messteil (9; 90) angeordneten Aperturhalters (30; 98) hindurchführbar ist, wobei das Messteil (9;90) mit der Entnahmevorrichtung verbunden ist,
- einer Aufnahmestation (III), in der als Wegwerfteile ausgebildete Messteile (9; 90) bereitstellbar und mit der Entnahmevorrichtung verbindbar sind, und
- einer Einrichtung zum Bewegen der Entnahmevorrichtung zwischen der Entnahmestation (I), der Messstation (II) sowie der Aufnahmestation (III), **dadurch gekennzeichnet, dass**
die Entnahmevorrichtung
- ein Nadelteil(15) aufweist, das mit einem in der Aufnahmestation (III) bereitgestellten Messteil (90) verbindbar ist oder
- ein Nadelteil (15) und ein Kopfteil (46) umfasst, das mit einem in der Aufnahmestation (III) bereitgestellten Messteil (9) verbindbar ist, wobei in der Entnahmestation (I) Blut in das Nadelteil (15) einführbar und in der Messstation (II) Blut durch das Kopfteil (46) dem Messteil (9) zuführbar ist, wobei ein Ventil (10) vorgesehen ist, um alternativ eine Verbindung zum Nadelteil (15) oder zum Kopfteil (46) herzustellen.

2. Einrichtung nach Anspruch 1, **dadurch gekennzeichnet, dass** die Einrichtung zum Bewegen der Entnahmevorrichtung einen durch einen ersten Antrieb (4) bewegbaren Arm (3) aufweist.

3. Einrichtung nach Anspruch 2, **dadurch gekennzeichnet, dass** der Arm (3) durch einen Schrittmotor zum Anfahren der Entnahmestation (I), der Messstation (II) und der Aufnahmestation (III) ansteuerbar ist.

4. Einrichtung nach einem der Ansprüche 1 bis 3, **dadurch gekennzeichnet, dass** der Arm (3) durch den ersten Antrieb (4) um eine Drehachse (2) drehbar ist und dass das Vorratsgefäß (40) der Entnahmestation (I) und eine Magazineinrichtung (7) für Messteile (9) der Aufnahmestation (II) jeweils durch einen ersten bzw. zweiten Radius von der Drehachse (2) beabstandet sind.

5. Einrichtung nach Anspruch 4, **dadurch gekennzeichnet, dass** das Nadelteil (15) und das Kopfteil (46) an dem Arm (3) entsprechend dem Abstand zwischen dem Vorratsgefäß (40) und der Magazineinrichtung (7) in Bezug auf die Drehachse (2) radial gegeneinander versetzt angeordnet sind

6. Einrichtung nach einem der Ansprüche 1 bis 3, **dadurch gekennzeichnet, dass** die Entnahmestation (I), die Messstation (II) und die Aufnahmestation (III) geradlinig nebeneinander angeordnet sind und dass der Arm (3) durch einen linear bewegbaren weiteren ersten Antrieb zu der Entnahmestation (I), der Messstation (II) bzw. der Aufnahmestation (III) bewegbar ist.

7. Einrichtung nach Anspruch 6, **dadurch gekennzeichnet, dass** der weitere erste Antrieb ein Schrittmotor ist, der zum Anfahren der Entnahmestation (I), der Messstation (II) sowie der Aufnahmestation (III) ansteuerbar ist.

8. Einrichtung nach einem der Ansprüche 1 bis 7, **dadurch gekennzeichnet, dass** an einem Auslegerteil (31) des Armes (3) ein zweiter Antrieb (11) zum Auf- und Abwärtsbewegen eines Halteteiles (13) angeordnet ist, wobei das Halteteil (13) die Entnahmevorrichtung umfasst.

9. Einrichtung nach Anspruch 8, **dadurch gekennzeichnet**, das der zweite Antrieb (11) einen Schrauben-Mutter-Mechanismus umfasst.

10. Einrichtung nach einem der Ansprüche 1 bis 9, **dadurch gekennzeichnet, dass** die Entnahmevorrichtung ein Nadelteil (15) aufweist, das an seiner dem Vorratsgefäß (40) abgewandten Seite mit einer wenigstens teilweise schlangen- oder spiralförmig am Halteteil (13) verlaufenden Rohrleitung (16) verbunden ist, die an ihrer dem Nadelteil (15) abgewandten Seite mit einer Leitung (14) in Verbindung steht, die zum Einsaugen von Blut über das Nadelteil (15) und die Rohrleitung (16) über ein
weiteres Ventil (18) mit einem Aufnahmereservoir (50) für Blut verbindbar ist, das durch einen dritten Antrieb (51) zum Einsaugen von Blut aus dem Vorratsgefäß (40) in das Aufnahmereservoir (50) oder zum Abgeben von Blut aus dem Aufnahmereservoir (50) in das Messteil (9;90) betätigbar ist.

11. Einrichtung nach Anspruch 10, **dadurch gekennzeichnet, dass** das Aufnahmereservoir (50) eine durch den dritten Antrieb (51) betätigbare Kolben/Zylindereinheit ist.

12. Einrichtung nach Anspruch 10 oder 11, **dadurch gekennzeichnet, dass** die Rohrleitung (16) und das Nadelteil (15) innen und/oder außenseitig hydrophob ausgebildet sind.

13. Einrichtung nach Anspruch 10 oder 11, **dadurch gekennzeichnet, dass** die Rohrleitung (16) und/oder das Nadelteil (15) aus einem innenseitig mit einer hydrophoben oder oleophoben Beschichtung versehenen Metallmaterial besteht.

14. Einrichtung nach Anspruch 13, **dadurch gekennzeichnet, dass** die Beschichtung eine Sol-Gel-Nanokomposit-Schicht oder eine teflonartige Fluorpolymer-Schicht ist.

15. Einrichtung nach einem der Ansprüche 8 bis 14, **dadurch gekennzeichnet, dass** das Halteteil (13) eine Heizeinrichtung (33) zum Aufwärmen des in die Rohrleitung (16) eingesaugten Blutes umfasst.

16. Einrichtung nach Anspruch 15, **dadurch gekennzeichnet, dass** die Heizeinrichtung (33) die Form eines metallischen Heizkernes besitzt, an dem die Rohrleitung (16) schlangen- oder spiralförmig angeordnet ist.

17. Einrichtung nach einem der Ansprüche 8 bis 16, **dadurch gekennzeichnet, dass** die Rohrleitung (16) derart schlangenförmig angeordnet ist, dass in aufeinanderfolgenden Schleifen der Rohrleitung (16) die Ober- und Unterseiten der über eine Biegung (161) jeweils verbundenen Abschnitte (162) der Rohrleitung (16) jeweils umgekehrt werden, so dass das eingesaugte Blut beim Durchlaufen der Abschnitte (162) zur Vermeidung einer Sedimentation senkrecht zur Strömungsrichtung gesehen in den aufeinanderfolgenden Abschnitten (162) quasi im Hinblick auf die Ober- und Unterseiten in den Abschnitten (162) umgekehrt wird.

18. Einrichtung nach einem der Ansprüche 10 bis 17, **dadurch gekennzeichnet, dass** in der Messstation (II) das Blut aus der Rohrleitung (16) über ein Messteil (90) durch Betätigen des dritten Antriebes (51) des Aufnahmereservoirs (50) durch die Apertur (26; 99) des Messteiles (9; 90) hindurchführbar ist, wobei das aus dem Messkopf (46) austretende Blut in einem Auffangbehälter (47) aufgefangen wird.

19. Einrichtung nach einem der Ansprüche 1 bis 18, **dadurch gekennzeichnet, dass** das Kopfteil (46) einen Bereich (23) aufweist, der dicht in eine Ausnehmung (21) des Messteiles (9) einführbar ist, dass im Kopfteil (46) eine mit der Rohrleitung (16) in Verbindung stehende Durchgangsöffnung (24) zu einer Vorkammer (27) im Bereich (23) verläuft, die sich am Ende des Bereiches (23) in Richtung auf die Bodenfläche (49) der Ausnehmung (21) des Messteiles (9) öffnet, wobei sich auf der Bodenfläche (49) der Aperturhalter (30) derart befindet, dass nach dem Einfahren des Bereiches (23) in die Ausnehmung (21) die Vorkammer (27) mit der Apertur (26) des Aperturhalters (30) in Verbindung steht, so dass über die Durchgangsöffnung (24) in die Vorkammer (27) befördertes Blut aus der Vorkammer (27) durch die Apertur (26) in einen Blutaustrittsraum (310) beförderbar ist, der im Messteil (9) an der der Vorkammer (27) abgewandten Seite der Apertur (26) angeordnet ist.

20. Einrichtung nach Anspruch 19, **dadurch gekennzeichnet, dass** die Abdichtung zwischen dem Kopfteil (46) und dem Messteil (9) durch eine O-Dichtung (22) erreicht wird, die an einer der Vorkammer (27) abgewandten Seite des Bereiches (23) gebildeten Schulter (470) anliegt und beim Einfahren des Bereiches (23) in die Ausnehmung (21) des Messteiles (9) an einer Fläche (48) des Randbereiches der Ausnehmung (21) dicht zur Anlage gelangt.

21. Einrichtung nach einem der Ansprüche 1 bis 18, **dadurch gekennzeichnet, dass** das Messteil (90) in einem Körperteil (91) eine Öffnung, vorzugsweise eine Bohrung (92) aufweist, die sich zu einer Seite des Körperteiles (91) öffnet, dass die Bohrung (92) im Körperteil (91) in eine Vorkammer (95) übergeht, die wiederum in einen erweiterten Raum (94) übergeht, wobei zwischen dem erweiterten Raum (94) und der Vorkammer (95) eine Schulter (97) besteht, an der der Aperturhalter (98) dicht anliegt, dass sich an den erweiterten Raum (94) eine sich zur anderen Seite des Körperteiles (91) öffnende Nachkammer (96) anschließt, wobei die Vorkammer (95) und die Nachkammer (96) über die Apertur (99) des Aperturhalters (98) in Verbindung stehen, und dass das Nadelteil (15) dicht in die Bohrung (92) einführbar ist.

22. Einrichtung nach Anspruch 21, **dadurch gekennzeichnet, dass** das Nadelteil (15) an seiner der Bohrung (92) zugewandten Seite einen als Einfahrschräge dienenden konischen Bereich (104) aufweist und dass die Bohrung (92) an ihrer dem Nadelteil (15) zugewandten Seite ein Dichtungselement (105) aufweist, das eine dichte Verbindung zwischen der Bohrung (92) und dem in diese eingefahrenen Nadelteil (15) herstellt.

23. Einrichtung nach Anspruch 22, **dadurch gekennzeichnet, dass** das Dichtungselement (105) eine O-Dichtung oder ein Dichtungswulst ist.

24. Einrichtung nach einem der Ansprüche 21 bis 23, **dadurch gekennzeichnet, dass** der Aperturhalter (98) an der Schulter (97) durch Ultraschall-Schweißen oder Kleben befestigt ist.

25. Einrichtung nach einem der Ansprüche 21 bis 24, **dadurch gekennzeichnet, dass** in der Bohrung (92) ein Röhrchen (93) dicht befestigt ist, über das Blut aus der Bohrung (92) zur Apertur (99) strömt.

26. Einrichtung nach Anspruch 25, **dadurch gekennzeichnet, dass** das Röhrchen (93) an der Innenwand der Bohrung (92) verklebt oder durch Flächenpressung befestigt ist.

27. Einrichtung nach Anspruch 25 oder 26, **dadurch gekennzeichnet, dass** der Außendurchmesser des Röhrchens (93) 0,8 bis 2,0 mm beträgt.

28. Einrichtung nach einem der Ansprüche 25 bis 27, **dadurch gekennzeichnet, dass** der Innendurchmesser des Röhrchens (93) 150 bis 500 µm beträgt.

29. Einrichtung nach einem der Ansprüche 25 bis 28, d**adurch gekennzeichnet**, dass das Röhrchen aus Edelstahl oder aus Fluor-Kunststoff besteht.

30. Einrichtung nach einem der Ansprüche 1 bis 29, **dadurch gekennzeichnet, dass** eine Spülstation (IV) vorgesehen und durch den Arm (3) anfahrbar ist, die ein ein Spülmittel enthaltenes Behältnis (42) umfasst, dem über eine Pumpe (19) Spülmittel entnehmbar ist, das zum Spülen über einen Leitungsabschnitt (142) der Leitung (14), der Rohrleitung (16) und der Entnahmevorrichtung (15) zuführbar ist.

31. Einrichtung nach einem der Ansprüche 1 bis 30, **dadurch gekennzeichnet, dass** in einer weiteren, durch den Arm (3) anfahrbaren Spülstation (V) das Nadelteil (15) und/oder gegebenenfalls der Messkopf (46) zur Reinigung der Außenseiten in ein ein Spül- oder Reinigungsmittel enthaltenes weiteres Behältnis (55) durch Betätigen des zweiten Antriebes (11) eintauchbar sind.

32. Verfahren zur automatischen Untersuchung von Blutproben mit einer Einrichtung nach einem der Ansprüche 8 bis 31, **dadurch gekennzeichnet, dass** die Einrichtung (3) zur Bewegung der Entnahmevorrichtung durch den ersten Antrieb (4) zur Entnahmestation (1) verfahren und der zweite Antrieb (11) in der Entnahmestation (I) zum Absenken des Halteteiles (13) und des Nadelteiles (15) betätigt werden, dass nach dem Eintauchen des Nadelteiles (15) in das im Vorratsgefäß (40) enthaltene Blut der dritte Antrieb (51) des Aufnahmereservoirs (50) derart betätigt wird, dass über das Nadelteil (15), das Rohrteil (16) und die Leitung (14) Blut in das Aufnahmereservoir (50) eingesaugt wird, wobei das weitere Ventil (18) eine Verbindung zwischen der Leitung (14) und dem Aufnahmereservoir (50) herstellt, dass
der Arm (3) zu Aufnahmestation (III) durch Betätigen des ersten Antriebes (4) verfahren wird, dass dem Magazin (7) zur Entnahme eines Messteiles (9; 90) das zuvor durch den zweiten Antrieb (11) nach oben verfahrene Halteteil (13) derart durch Betätigen des zweiten Antriebes (11) abgesenkt wird, dass ein Messteil (9; 90) mit dem Kopfteil (46) oder mit dem Nadelteil (15) dicht verbunden wird, dass
nach der Aufnahme des Messteiles (9; 90) der zweite Antrieb (11) zur Bewegung des Halteteiles (13) und zur Entnahme des Messteiles (9; 90) aus der Magazineinrichtung (7) betätigt wird, dass
der Arm (3) durch den ersten Antrieb (4) zur Messstation (II) bewegt wird, wobei das Aufnahmereservoir(50) durch den dritten Antrieb (51) so betätigt wird, dass ständig Blut durch die Apertur (26) des Messteiles (9; 90) strömt und dieses durch den Blutaustrittsraum (31) verlässt.

33. Verfahren nach Anspruch 32, **dadurch gekennzeichnet, dass** vor der Ausführung einer Messoperation eine Spülflüssigkeit, vorzugsweise eine physiologische NaCl-Lösung, aus einem Behältnis (42) in die Leitung (142) und über das weitere Ventil (18) in einer ersten Stellung desselben in die Rohrleitung (16) und das Nadelteil (15) befördert wird, dass danach Blut aus dem Vorratsgefäß (40) eingesaugt wird, derart, dass zwischen dem Blut und der Spülflüssigkeit eine Luftblase (44) besteht, dass bis zur Vornahme einer Messoperation das Blut und die Spülflüssigkeit in der ersten Stellung des ersten Ventils(18) in der Leitung (142) und der Rohrleitung (16) hin- und herbewegt werden, um eine Sedimentierung des Blutes zu vermeiden, und dass zur Ausführung einer Messoperation über das weitere Ventil (18) in einer zweiten Stellung desselben eine Verbindung zwischen dem Blut und dem Aufnahmereservoir (50) zum Befüllen desselben mit Blut hergestellt wird.

## Claims

1. Apparatus for automatically analyzing blood samples, comprising
- an extractor station (I) in which blood may be removed from a reservoir (40) with the aid of extraction means,
- a measuring station (II) in which the extracted blood may be passed through an aperture (26; 99) in an aperture holder (30; 98) located in a measuring part (9; 90), said measuring part (9; 90) being coupled to the extraction means,
- a receiving station (III) in which measuring parts (9, 90) may be provided and coupled to said extraction means, said measuring parts configured to be disposable; and
- means for moving said extraction means between extractor station (I), measuring station (II) and receiving station (III),
**characterized in that** said extraction means comprises
- a needle part (15) adapted to be coupled to a measuring part (90) provided in receiving station (III), or
- a needle part (15) and head part (46) adapted to be coupled to a measuring part (9) provided in receiving station (III),
with blood being conveyable in measuring station (II) through head part (46) to measuring part (9), and with a valve (10) being provided to allow an alternative connection to be established to needle part (15) or to head part (46).

2. Apparatus according to claim 1, **characterized in that** said means for moving the extraction means includes an arm (3) adapted to be moved by first drive means (4).

3. Apparatus as in claim 2, **characterized in that** arm (33) is adapted to be controlled by a stepper motor to be moved towards extractor station (I), measuring station (II) and receiving station (III).

4. Apparatus as in any one of claims 1 to 3, **characterized in that** arm (3) is rotatable about a first axis of rotation (2) by said first drive means (4) and **in that** reservoir (40) of extractor station (I), as well as magazine means (7) for measuring part (9) of receiving station (II), are spaced by a first and a second radius, respectively, from said axis of rotation (2).

5. Apparatus as in claim 4, **characterized in that** needle part (15) and head part (46) are disposed on arm (3) in accordance with the distance between reservoir (40) and magazine means (7) in a radially offset relationship relative to axis of rotation (2).

6. Apparatus as in any one of claims 1 to 3, **characterized in that** extractor station (I), measuring station (II) and receiving station (III) are disposed in a straight line one behind the other and **in that** arm (3) is movable towards extractor station (I), measuring station (II) and receiving station (III), respectively, by additional first drive means, said latter drive means being movable in a straight line.

7. Apparatus as in claim 6, **characterized in that** said additional first drive means comprises a stepper motor adapted to be controlled for movement towards extractor station (I), measuring station (II) and receiving station (III).

8. Apparatus as in any one of claims 1 to 7, **characterized in that** a beam portion (31) of arm (3) has thereon second drive means (11) for the up-and-down reciprocation of a holding member (13), said holding member (13) encircling said extraction means.

9. Apparatus as in claim 8, **characterized in that** second drive means (11) comprises a screw-and-nut mechanism.

10. Apparatus as in any one of claims 1 to 9, **characterized in that** said extraction means comprises a needle part (15) connected at its side opposite reservoir (40) with a tubing (16) run along holding member (13) at least partly in a serpentine or spiraling pattern thereon, **in that** said tubing is connected on its side opposite needle part (15) with a duct (14) adapted to be connected for the aspiration of blood through needle part (15) and tubing (16) through another valve (18) with a reservoir (50) for the reception of blood, and **in that** said other valve (18) is actuatable by third drive means (51) for the aspiration of blood from reservoir (40) into receiving reservoir (50) or for the discharge of blood from receiving reservoir (50) into measuring part (9; 90).

11. Apparatus as in claim 10, **characterized in that** receiving reservoir (50) comprises a piston-cylinder unit adapted to be actuated by said third drive means (51).

12. Apparatus as in claim 10 or 11, **characterized in that** tubing (16) and needle part (15) are configured to be hydrophobic on the inside and/or on the outside.

13. Apparatus as in claim 10 or 11, **characterized in that** tubing (16) and/or needle part (15) consist(s) of a metal material provided with a hydrophobic or oleophobic coating on the inside.

14. Apparatus as in claim 13, **characterized in that** said coating comprises a sol-gel-nanocomposite layer or a teflon-like fluoropolymer layer.

15. Apparatus as in any one of claims 8 to 14, **characterized in that** holding member (13) comprises heater means (33) for heating the blood aspirated into tubing (16).

16. Apparatus as in claim 15, **characterized in that** heater means (33) is in the form of a metal heating core on which tubing line (16) is disposed in a serpentine or spiral pattern.

17. Apparatus as in any one of claims 8 to 16, **characterized in that** tubing (16) is disposed in a serpentine pattern so as to reverse the top and bottom sides of sections (162) in the consecutive loops of tubing (16), whereby, to avoid sedimentation, the aspirated blood, when flowing through sections (162), and seen in a direction perpendicular to the direction of flow, is reversed in said consecutive sections (162) relative to the top and bottom sides thereof.

18. Apparatus as in any one of claims 10 to 17, **characterized in that** the blood from tubing (16) is conveyable in measuring station (II) through a measuring part (9; 90) via aperture (26; 99) of the latter by acutating said third drive means (51) of receiving reservoir (50), with the blood discharged from measuring head (46) being collected in a vessel (47).

19. Apparatus as in any one of claims 1 to 18, **characterized in that** head part (46) has a portion (23) adapted to be introduced tight in a recess (21) of measuring part (9), **in that** head part (46) has there through in area (23) an opening (24) to an ante-chamber (27), said opening extending at the end of area (23) in a direction towards bottom surface (49) of recess (21) of measuring part (9), with aperture holder (30) disposed on bottom surface (49) so that, following the introduction of area (23) into recess (21), ante-chamber (27) communicates with aperture (26) of aperture holder (30) so that blood conveyed through opening (24) into ante-chamber (27) may be conveyed from ante-chamber (27) through aperture (26) into a blood outlet space (310) in measuring part (9) on the side of aperture (26) facing away from ante-chamber (27).

20. Apparatus as in claim 19, **characterized in that** the seal between head part (46) and measuring part (9) is effected by an O-ring (22) supported on a shoulder (470) formed on the side of area (23) facing away from ante-chamber (27), said O-ring sealingly engaging a surface (48) of the edge portion of recess (21) as area (23) enters recess (21) of measuring part (9).

21. Apparatus as in any one of claims 1 to 18, **characterized in that** measuring part (90) has in a portion (91) of the body thereof an opening, preferably a borehole (92), opening towards one side of body portion (91); **in that** borehole (92) in body portion (91) opens into an ante-chamber (95) which in turn opens into an expanded space (94), with a shoulder (97) formed between expanded space (94) and ante-chamber (95) which sealingly engages aperture holder (98); **in that** said expanded space (94) merges with a post-chamber (96) opening towards the other side of body portion (91), with ante-chamber 95) and post-chamber (96) in fluid communication via aperture (99) of aperture holder (98); and **in that** needle part (15) is sealingly insertable into borehole (92).

22. Apparatus as in claim 21, **characterized in that** needle part (15) has on its side facing borehole (92) a conical portion (104) serving as slanted insertion guide means; and **in that** borehole (92) has on its side facing needle part (15) a sealing element (105) ensuring a sealed engagement of needle part (15) introduced in borehole (92).

23. Apparatus as in claim 22, **characterized in that** sealing element (105) comprises an O-ring or a sealing ridge.

24. Apparatus as in any one of claims 21 to 23, **characterized in that** aperture holder (98) is secured to shoulder (97) by bonding or ultrasonic welding.

25. Apparatus as in any one of claims 21 to 24, **characterized in that** borehole (92) has sealingly secured therein a small tube (93) through which the blood flows from borehole (92) to aperture (99).

26. Apparatus as in claim 25, **characterized in that** tube (93) is secured to the inner walls of borehole (92) by bonding or by contact pressure.

27. Apparatus as in claim 25 or 26, **characterized in that** the outer diameter of tube (93) is 0.8 mm to 2.0 mm.

28. Apparatus as in any one of claims 25 to 27, **characterized in that** the inner diameter of tube (93) is 150 µm to 500 µm.

29. Apparatus as in any one of claims 25 to 28, **characterized in that** said tube comprises high-grade steel or a fluoro plastics material.

30. Apparatus as in any one of claims 1 to 29, **characterized by** a rinsing station (IV) to which arm (3) is adapted to travel, said rinsing station comprising a receptacle (42) holding a rinse agent and adapted to have rinse agent extracted therefrom by a pump (19) to be conveyed for rinsing through a duct section (142) to duct (14), tubing (16) and extraction means (15).

31. Apparatus as in any one of claims 1 to 30, **characterized by** another rinsing station (V) to which arm (3) is adapted to travel, wherein needle part (15) and/or measuring head (46) are adapted to be immersed for the exterior cleaning thereof in an additional receptacle (55) holding a rinsing or cleaning agent, such immersion being effected by actuating second drive means (11).

32. A process for the automatic analysis of blood samples by means of apparatus as defined in any one of claims 8 to 31, **characterized in that** means (3) for moving the extraction means is moved by said first drive means (4) to extractor station (I) and second drive means (11) is actuated in extractor station (I) for lowering holding part (13) and needle part (15); **in that** said third drive means (51) of receiving reservoir (50) is actuated, following the immersion of needle part (15) into the blood held in reservoir (40), in such a manner that blood is aspirated into receiving reservoir (50) via needle part (15), tubing (16) and duct (14), said additional valve (18) establishing a connection between duct (14) and receiving reservoir (50); **in that** arm (3) is caused to travel to receiving station (III) by actuating first drive means (4), with magazine (7), after having been moved upwards by second drive means (11), being lowered for the removal of a measuring part (9; 90) by actuating second drive means (11) in such a manner that a measuring part (9; 90) is sealingly coupled to head part (46) or to needle part (15); **in that**, following the picking up of measuring part (9; 90), said second drive means (11) is actuated to move holding member (13) and to withdraw measuring part (9; 90) from magazine means (7); and **in that** arm (3) is caused by first drive means (4) to travel to measuring station (II), with receiving reservoir (50) actuated by third drive means (51) so that blood flows continuously through aperture (26) of measuring part (9; 90) and exits from it through blood discharge space (31).

33. Process as in claim 32, **characterized in that**, prior to a measuring operation, a rinse fluid - preferably a physiological saline solution - is conveyed from a receptacle (42) into duct (142) and via said additional valve (18) in first position thereof into tubing (16) and into needle part (15); **in that** blood is then extracted from reservoir (40) in a manner such that an air bubble (44) remains between the blood and the rinse fluid; **in that**, until a measuring operation is performed, the blood and the rinse fluid are moved back and forth in duct (142) and in tubing (16) in the first position of first valve (18) to avoid sedimentation of the blood; and **in that**, for a measuring operation to be performed, a connection between the blood and the receiving reservoir (50) is established through additional valve (18) so as to fill said reservoir with blood.

## Revendications

1. Dispositif d'analyse automatique d'échantillons de sang, comportant
- une station de prélèvement (I) dans laquelle le sang peut être prélevé depuis un réservoir de réserve (40) au moyen d'un dispositif de prélèvement,
- une station de mesure (II) dans laquelle on peut faire passer du sang prélevé à travers un orifice (26 ; 99) d'un support à orifices (30 ; 98) agencé dans un élément de mesure (9 ; 90), l'élément de mesure (9 ; 90) étant reliée au dispositif de prélèvement,
- une station de réception (III) dans laquelle des éléments de mesure (9 ; 90) réalisés sous forme de pièces jetables peuvent être mis à disposition et reliés avec le dispositif de prélèvement, et
- un dispositif pour déplacer le dispositif de prélèvement entre la station de prélèvement (I), la station de mesure (II) ainsi que la station de réception (III), **caractérisé en ce que**
le dispositif de prélèvement
- présente une aiguille (15) qui peut être reliée à un élément de mesure (90) mis à disposition dans la station de réception (III) ou
- comprend une aiguille (15) et une tête (46) qui peut être reliée avec un élément de mesure (9) mis à disposition dans la station de réception (III), du sang pouvant, dans la station de prélèvement (I), être introduit dans l'aiguille (15) et pouvant, dans la station de mesure (II), être amené par la tête (46) à l'élément de mesure (9), une soupape (10) étant prévue pour établir une liaison alternativement vers l'aiguille (15) ou vers la tête (46).

2. Dispositif selon la revendication 1, **caractérisé en ce que** le dispositif présente un bras (3) mobile grâce à un premier entraînement (4) pour déplacer le dispositif de prélèvement.

3. Dispositif selon la revendication 2, **caractérisé en ce que** le bras (3) peut être piloté par un moteur pas-à-pas pour aborder la station de prélèvement (I), à la station de mesure (II) et la station de réception (III).

4. Dispositif selon l'une des revendications 1 à 3, **caractérisé en ce que** le bras (3) est rotatif autour d'un axe de rotation grâce au premier entraînement (4), et **en ce que** le réservoir de réserve (40) de la station de prélèvement (I) et un dispositif formant magasin (7) pour des éléments de mesure (9) de la station de réception (III) sont espacés chacun de l'axe de rotation (2) par un premier et par un deuxième rayon, respectivement.

5. Dispositif selon la revendication 4, **caractérisé en ce que** l'aiguille (15) et la tête (46) sont agencées avec décalage mutuel radial par rapport à l'axe de rotation (2) sur le bras (3) en correspondance avec l'espacement entre le réservoir de réserve (40) et le dispositif à magasin (7).

6. Dispositif selon l'une des revendications 1 à 3, **caractérisé en ce que** la station de prélèvement (I), la station de mesure (II) et la station de réception (III) sont agencées en ligne droite les unes à côté des autres, et **en ce que** le bras (3) est mobile grâce à un autre premier entraînement linéairement mobile vers la station de prélèvement (I), la station de mesure (II) et la station de réception (III).

7. Dispositif selon la revendication 6, **caractérisé en ce que** l'autre premier entraînement est un moteur pas-à-pas qui peut être piloté pour aborder la station de prélèvement (I), la station de mesure (II) et la station de réception (III).

8. Dispositif selon l'une des revendications 1 à 7, **caractérisé en ce que** sur un élément formant flèche (31) du bras (3) est agencé un deuxième entraînement (11) pour faire monter et descendre un élément de retenue (13), l'élément de retenue (13) comprenant le dispositif de prélèvement.

9. Dispositif selon la revendication 8, **caractérisé en ce que** le deuxième entraînement (11) comprend un mécanisme à vis et écrous.

10. Dispositif selon l'une des revendications 1 à 9, **caractérisé en ce que** le dispositif de prélèvement présente une aiguille (15) qui peut être reliée, sur sa face détournée du réservoir de réserve (4), avec une conduite tubulaire (16) s'étendant au moins partiellement en forme de serpentin ou de spirale sur l'élément de retenue (13), laquelle est en communication, sur sa face détournée de l'aiguille (15), avec une conduite (14) qui, pour aspirer du sang via l'aiguille (15) et la conduite tubulaire (16), peut être reliée avec un réservoir de réception (50) de sang via une autre soupape (18) qui peut être actionnée par un troisième entraînement (51) pour aspirer du sang hors du réservoir de réserve (40) dans le réservoir de réception (50) ou pour délivrer dans l'élément de mesure (9 ; 90) du sang provenant du réservoir de réserve (40).

11. Dispositif selon la revendication 10, **caractérisé en ce que** le réservoir de réception (50) est une unité à piston et cylindre actionnable par le troisième entraînement (51).

12. Dispositif selon la revendication 10 ou 11, **caractérisé en ce que** la conduite tubulaire (16) et l'aiguille (15) sont réalisés hydrophobes à l'intérieur et/ou sur la face extérieure.

13. Dispositif selon la revendication 10 ou 11, **caractérisé en ce que** la conduite tubulaire (16) et/ou l'aiguille (15) sont/est constituée par un matériau métallique pourvu d'un revêtement hydrophobe ou oléophobe sur sa face intérieure.

14. Dispositif selon la revendication 13, **caractérisé en ce que** le revêtement est une couche sol-gel nanocomposite ou une couche de polymère fluoré de type téflon.

15. Dispositif selon l'une des revendications 8 à 14, **caractérisé en ce que** l'élément de retenue (13) est un dispositif de chauffage (33) pour chauffer le sang aspiré dans la conduite tubulaire (16).

16. Dispositif selon la revendication 15, **caractérisé en ce que** le dispositif de chauffage (33) possède la forme d'un noyau chauffant sur lequel la conduite tubulaire (16) est agencée en forme de serpentin ou de spirale.

17. Dispositif selon l'une des revendications 8 à 16, **caractérisé en ce que** la conduite tubulaire (16) est agencée en forme de serpentin de telle sorte que dans des boucles successives de la conduite tubulaire (16), les faces supérieures et inférieures des tronçons (162) de la conduite tubulaire (16), lesquels sont reliés chacun via un méandre (161), sont chacune renversées, de telle sorte que pour éviter une sédimentation, le sang aspiré est quasiment renversé lorsqu'il passe à travers les tronçons (162), vu perpendiculairement à la direction d'écoulement, au niveau des faces supérieures et inférieures dans les tronçons (162) qui se succèdent.

18. Dispositif selon l'une des revendications 10 à 17, **caractérisé en ce que** dans la station de mesure (II), le sang provenant de la conduite tubulaire (16), peut passer à travers l'orifice (26 ; 99) de l'élément de mesure (9 ; 90) via un élément de mesure (9 ; 90) en actionnant le troisième entraînement (51) du réservoir de réception (50), le sang sortant de la tête de mesure (46) étant recueilli dans un récipient collecteur (47).

19. Dispositif selon l'une des revendications 1 à 18, **caractérisé en ce que** la tête (46) présente une zone (23) qui peut être introduite de manière étanche dans un évidement (21) de l'élément de mesure (9), **en ce que** dans la tête (46), une ouverture de passage (24) en liaison avec la conduite tubulaire (16) s'étend dans la zone (23) vers une chambre préliminaire (27), ouverture qui s'ouvre à l'extrémité de la zone (23) en direction de la surface de fond (49) de l'évidement (21) de l'élément de mesure (9), le support d'orifice (30) se trouvant sur la surface de fond (49) de telle sorte qu'après que la zone (30) entre dans l'évidement (21), la chambre préliminaire (27) est en communication avec l'orifice (26) du support d'orifice (30), de telle sorte que du sang transporté via l'ouverture de passage (24) jusque dans la chambre préliminaire (27) peut être transporté hors de la chambre préliminaire (27) à travers l'orifice (26) jusque dans un espace de sortie de sang (310) qui est agencé dans l'élément de mesure (9) sur la face de l'orifice (26) qui est détournée de la chambre préliminaire (27).

20. Dispositif selon la revendication 19, **caractérisé en ce que** l'étanchement entre la tête (46) et l'élément de mesure (9) est obtenue par un joint torique qui est en appui sur un épaulement (470) formé sur la face de la zone (23), qui est détournée de la chambre préliminaire (27) et qui, lors de l'entrée de la zone (23) dans l'évidement (21) de l'élément de mesure (9), parvient en appui étanche sur une surface (48) de la région de bord de l'évidement (21).

21. Dispositif selon l'une des revendications 1 à 18, **caractérisé en ce que** l'élément de mesure (90) présente, dans une partie de corps (91), une ouverture, de préférence un perçage (92), qui s'ouvre sur un côté de la partie de corps (91), **en ce que** le perçage (92) dans la partie de corps (91) se transforme en une chambre préliminaire (95) qui se transforme à son tour en un espace (94) élargi, un épaulement (97) étant formé entre l'espace élargi (94) et la chambre préliminaire (97), épaulement sur lequel le support d'orifice (98) est en appui étanche, **en ce qu'**à l'espace élargi (94) se raccorde une chambre postérieure (96) s'ouvrant sur l'autre côté de la partie de corps (91), la chambre préliminaire (95) et la chambre postérieure (96) étant en communication via l'orifice (99) du support d'orifice (98), et **en ce que** l'aiguille (15) peut être introduite de manière étanche dans le perçage (92).

22. Dispositif selon la revendication 21, **caractérisé en ce que** sur sa face tournée vers le perçage (92), l'aiguille (15) présente une zone conique (104) servant de pente d'introduction, et **en ce que** sur sa face tournée vers l'aiguille (15), le perçage (92) présente un élément d'étanchéité (105) qui établit une liaison étanche entre le perçage (92) et l'aiguille (15) entrant dans celui-ci.

23. Dispositif selon la revendication 22, **caractérisé en ce que** l'élément d'étanchéité (105) est un joint torique ou un bourrelet d'étanchéité.

24. Dispositif selon l'une des revendications 21 à 23, **caractérisé en ce que** le support d'orifice (98) est fixé sur l'épaulement (97) par soudure à ultrasons ou par collage.

25. Dispositif selon l'une des revendications 21 à 24, **caractérisé en ce que** dans le perçage (92) est fixé de manière étanche un tube (93) via lequel le sang s'écoule depuis le perçage (92) vers l'orifice (99).

26. Dispositif selon la revendication 25, **caractérisé en ce que** le tube (93) est collé ou fixé par pressage surfacique sur la paroi intérieure du perçage (92).

27. Dispositif selon les revendications 25 ou 26, **caractérisé en ce que** le diamètre extérieur du tube (93) est de 0,8 à 2,0 mm.

28. Dispositif selon l'une des revendications 25 à 27, **caractérisé en ce que** le diamètre intérieur du tube (93) est de 150 à 500 µm.

29. Dispositif selon l'une des revendications 25 à 28, **caractérisé en ce que** le tube est en acier spécial ou en matière plastique fluorée.

30. Dispositif selon l'une des revendications 1 à 29, **caractérisé en ce qu'**il est prévu une station de rinçage (IV) qui peut être abordée par le bras (3), laquelle comprend un réceptacle (42) contenant un produit de rinçage et hors duquel on peut soutirer, via une pompe (19), du produit de rinçage qui peut être amené à la conduite (14), à la conduite tubulaire (16) et au dispositif de prélèvement (15) via un tronçon de conduite (142).

31. Dispositif selon l'une des revendications 1 à 30, **caractérisé en ce que** dans une autre station de rinçage (V) abordable par le bras (3), l'aiguille (15) et/ou, le cas échéant, la tête de mesure (46) peuvent être plongées dans un autre réceptacle (55) contenant un produit de rinçage ou de nettoyage, par actionnement du deuxième entraînement (11), pour nettoyer les faces extérieures.

32. Procédé d'analyse automatique d'échantillons de sang comportant un dispositif selon l'une des revendications 8 à 31, **caractérisé en ce que** le dispositif (3) destiné à déplacer le dispositif de prélèvement est amené par le premier entraînement (4) à la station de prélèvement (1), et le deuxième entraînement (11) est actionné dans la station de prélèvement (I) pour faire descendre l'élément de retenue (13) et l'aiguille (15), **en ce qu'**après avoir plongé l'aiguille (1) dans le sang contenu dans le réservoir de réserve (40), le troisième entraînement (51) du réservoir de réception (50) est actionné de telle sorte que du sang est aspiré via l'aiguille (15), la conduite tubulaire (16) et la conduite (14) jusque dans le réservoir de réception (50), l'autre soupape (18) établissant une communication entre la conduite (14) et le réservoir de réception (50),
**en ce que** le bras (3) est amené à la station de réception (III) par actionnement du premier entraînement (4), **en ce que** pour prélever un élément de mesure (9 ; 90) hors du magasin, l'élément de retenue (13) amené auparavant vers le haut par le deuxième entraînement (11) est descendu par actionnement du deuxième entraînement (11) de telle sorte qu'un élément de mesure (9 ; 90) est relié de manière étanche avec la tête (46) ou avec l'aiguille (15),
**en ce qu'**après la réception de l'élément de mesure (9 ; 90), le deuxième entraînement (11) est actionné pour déplacer l'élément de retenue (13) et pour retirer l'élément de mesure (9 ; 90) hors du magasine (7),
**en ce que** le bras (3) est amené par le premier entraînement (4) jusqu'à la station de mesure (II), le réservoir de réception (50) étant actionné par le troisième entraînement (51) de telle sorte que du sang s'écoule en permanence à travers l'orifice (26) de l'élément de mesure (9 ; 90) et que celui-ci quitte l'espace de sortie de sang (31).

33. Procédé selon la revendication 32, **caractérisé en ce que** avant d'effectuer une opération de mesure, un liquide de rinçage, de préférence une solution de NaCl physiologique, est transportée depuis un réceptacle (42) dans la conduite (142) et, via l'autre soupape (18), dans une première position de celle-ci, dans la conduite tubulaire (16) et dans l'aiguille (15), **en ce qu'**ensuite, du sang est aspiré hors du réservoir de réserve (40), de telle sorte qu'une bulle d'air (44) se trouve entre le sang et le liquide de rinçage, **en ce que** jusqu'à ce qu'on effectue une opération de mesure, le sang et le liquide de rinçage sont agités, dans la première position de la première soupape (18), dans la conduite (42) et dans la conduite tubulaire (16), et **en ce que** pour effectuer une opération de mesure via l'autre soupape (18) dans une deuxième position de celle-ci, une liaison est établie entre le sang et le réservoir de réception (50) pour remplir celui-ci avec du sang.
